# EUROPEAN PATENT SPECIFICATION

(11) **EP 1 592 463 B1**
(45) Date of publication and mention of the grant of the patent: **16.08.2006**
(21) Application number: 03701418.0
(22) Date of filing: 13.02.2003
(51) Int. Cl.: A61L 24/00

(54) **INJECTABLE BONE-REPLACEMENT MIXTURE**
INJIZIERBARE KNOCHENERSATZMISCHUNG
MELANGE INJECTABLE DE REMPLACEMENT D'OS

(43) Date of publication of application: 09.11.2005
(73) Proprietor: Synthes AG Chur, 7002 Chur (CH)
(72) Inventor: BISIG, Adrian, CH-7270 Davos (CH); BOHNER, Marc, CH-2540 Grenchen (CH); SCHNEIDER, Erich, CH-7270 Davos (CH)
(74) Representative: Lusuardi, Werther
(86) International application number: PCT/CH2003/000105
(87) International publication number: WO 2004/071543

(56) References cited:
- WO-A-99/62570
- US-A- 4 093 576
- BRUENS MARCO L ET AL: "Porous polymethylmethacrylate as bone substitute in the craniofacial area." THE JOURNAL OF CRANIOFACIAL SURGERY. UNITED STATES JAN 2003, vol. 14, no. 1, January 2003 (2003-01), pages 63-68, XP009008805 ISSN: 1049-2275
- DEB S ET AL: "Radiopacity in bone cements using an organo-bismuth compound" BIOMATERIALS, ELSEVIER SCIENCE PUBLISHERS BV., BARKING, GB, vol. 23, no. 16, August 2002 (2002-08), pages 3387-3393, XP004359626 ISSN: 0142-9612

## Description

The invention relates to an injectable mixture for substituting bone tissue in situ, in particular for bone augmentation, such as vertebroplasty, femoroplasty (femoral neck augmentation), humeroplasty (humerus head augmentation) according to the preamble of claim 1.

Polymethylmethacrylate (PMMA) bone cement is by far the most frequently used material known in the art of bone augmentation (e.g. percutaneous vertebroplasty). However, there are serious complications in the use of this material such as cement leakage, monomer toxicity, necrosis, and increased fracture rate of the adjacent vertebrae.

By "cement leakage" is meant the leakage of the injected cement paste out of the bone, in particular into the spinal canal, which can provoke neurological damages such as paralysis. The injected cement can also go into blood vessels and provoke an embolism.

As is well known PMMA cement hardens according to a very exothermic reaction. Therefore, the tissues surrounding the injected cement might become heated up at temperatures high enough to provoke tissue necrosis.

The increased fracture rate mentioned above is caused by an inadequate stiffness of the augmented segment within an osteoporotic spine and results from the fact that PMMA cement is much stiffer than cancellous bone. Therefore, the whole biomechanical stability of the vertebrae is modified by the presence of the PMMA cement. These biomechanical changes lead to an increased incidence of fractures of the vertebrae adjacent to the augmented vertebrae. The possible countermeasure of prophylactic augmentation of the adjacent levels has the drawback that it enlarges the intervention and enhances the risk for additional cement leakage.

From US-A-4 093 576 deWijn it is known to mix a doughy bone cement mixture with a highly viscous aqueous gel to form a dispersion of the bone cement with the gel. This mixture is used for anchoring prosthetic joints into bone, namely to increase the bone-cement interface for an increased anchorage of the prosthesis. Since the gel is water soluble, it will be washed out after implantation in the body leaving back a porous bone cement. One of the major drawbacks of the material according to US-A-4 093 576 deWijn is the use of metallic ions as X-ray contrasting agent. Such particles are incorporated into the gel and therefore, these particles are washed away and can provoke compatibility problems.

In the materials according to the state of the art which use inorganic X-ray contrast agent, like zirconium dioxide and barium sulfate in solid particle form there is a phase separation between the MMA and the inorganic, solid X-ray contrast agent. This is probably caused because of the hydrophilic properties of the heavy metal ions in combination with the hydrophobic properties of the PMMA. If water is used as third component in the mixture the inorganic X-ray contrast agent selectively accumulate into the aqueous phase. Therefore, complications may occur for clinical applications because of the washing-out of the aqueous phase. Clinical follow-up is not possible because of the lacking radio-opacity after a certain time of washing-out.

Inorganic X-ray contrast agents (BaSO₄, ZrO₂) selectively accumulate into the aqueous phase and thus are washed-out into the blood circulation within a few days with the risk of embolism and toxic reactions. In this context it has to be observed that he amount of X-ray contrast agent necessary for the injection control in bone augmentation is very large, i.e. much larger than for other applications such as the fixation of hip prosthesis for example according to US-A-4 093 576 deWijn. Washing out of such a large portion of inorganic heavy metal ions in the patient may be very dangerous or even perilous.

On this point, the invention intends to provide remedial measures. The invention is based on the objective of providing an injectable self-hardening mixture which upon hardening a subsequent washing out of material in situ results in a porous bone substitute material having a reduced stiffness compared to a conventional hardened PMMA bone cement and which has an optimal radio-opacity.

The invention solves the posed problem with a mixture that displays the features of claim 1 as well as with the use of such as mixture that displays the features of claims 33 and a method for preparing such an injectable mixture that displays the features of claim 38.

The injectable bone substitute material for bone augmentation has adaptable mechanical properties, an optimal radio-opacity without any inorganic X-ray contrast agent and therefore good biocompatibility.

The advantages achieved by the invention are essentially to be seen in the fact that, thanks to the mixture according to the invention:
a) An optimal biocompatibility is achieved by using an organic X-ray contrast agent, preferably one certified for parenteral application. Organic X-ray contrast agents (preferably iodine-containing) can be prepared as an aqueous solution and therefore can directly replace the entire aqueous phase. To allow a follow-up control after washing-out of the aqueous phase preferably a lipophilic X-ray contrast agent can be used additionally with selective accumulation into the PMMA phase;
b) The high radiopacity of the injectable mixture makes it clearly radiologically visible so that cement extravasation during injection can be prevented;
c) The stiffness of the bone substitute material obtained in situ is reduced and adaptable to the properties of osteoporotic bone, hence the risk of fractures of the vertebrae adjacent to the augmented vertebra is lower;
d) A lower amount of polymerization heat is released during polymerization; hence the risk of bone necrosis is lower;
e) The optimal handling and extensive experiences in the application of PMMA based powder/liquid bone cements for vertebroplasty can be utilized.

In a preferred embodiment the X-ray contrast agent is a liquid substance or a solid substance dissolved in a liquid solvent, preferably in water. The X-ray contrast agent may be based on iodine and preferably is chosen from the following group of substances: iopromidum, iopamidol, aminotrizoate acid, iotroxin acid, iopodin acid, iomeprol, iodamid, ioxithalamate, iothalamate, ioxaglin acid and lipiodol (iodised ethyl ester of the fatty acids of poppy-seed oil).

The iodine-based X-ray contrast agent may be used in an aqueous solution, preferably in a concentration of 30 to 80 weight % . The injectable mixture may comprises at least 5 weight %, preferably at least 20 weight % of said X-ray contrast agent.

In a further embodiment the viscosity of said third component is lower than 200 Pa.s (200,000 centipoise). The viscosity of said third component may be lower than 100 Pa.s (100,000 centipoise), preferably lower than 20 Pa.s (20,000 centipoise). 300 Pa.s

In a further embodiment the viscosity of said third component purposefully is comprised between 1 and 100 Pa.s (1,000 and 100,000 centipoise) preferably between 2 and 50 Pa.s (2,000 and 50,000 centipoise).

In a further embodiment the viscosity of the injectable mixture measured 4 minutes after mixing of all components is in the range of 200 to 300 Pa.s (200,000 to 300,000 centipoise). Below 200 Pa.s (200,000 centipoise) the injected mixture tends to leak from the treated bone; above 300 Pa.s (300,000 centipoise) the force required to inject the mixture becomes rapidly too large to enable manual injection.

In a preferred embodiment said two-component bone cement is based on a polyacrylic cement (in particular a polymethacrylic cement) or a calcium phosphate cement. Said two-component bone cement is preferably a powder/liquid system base on polymethylmethacrylate (PMMA) powder and monomethylmethacrylate (MMA) liquid with a polymerization catalyst and a polymerization accelerator.

The third component may comprise water and discrete particles of a water-soluble solid substances. Said water-soluble solid substance may be taken from the group of polysaccharides, in particular: chondroitin sulfate, carboxymethyl cellulose, hydroxyethylmethylcellulose, fucan, carregeenan, dextran, heparin, heparan sulfate, hydroxyethtycellulose (HEC), hydroxypropylmethyl cellulose, sodium alginate, chitosan or a hyaluronate.

In a further embodiment said third component is an aqueous hyaluronate solution with a concentration of 0.1% to 5.0%, preferably of 1.0 % to 2.0 %. Typically the concentration may by 0.5 %.

The molecular weight of said hyaluronate should be at least 500,000 Daltons, preferably at least 800,000 Daltons. The molecular weight of said hyaluronate should be below 5,000,000 Daltons, preferably below 2,000,000 Daltons. Typically the molecular weight of the hyaluronate used is 1,100,000 Daltons.

Said water-soluble solid substance may be taken from the group of gelatin or collagen.

In a further embodiment said third component is a hydrophobic liquid which preferably is selected from the group of:
ricinoleic acid (C₁₇H₃₃OCOOH), linoleic acid (C₁₇H₃₁COOH), palmitic acid (C₁₅H₃₁COOH), palmitoleic acid (C₁₅H₂₉COOH), stearic acid (C₁₇H₃₅COOH), linolenic acid (C₁₇H₂₉COOH), arachidic acid (C₁₉H₃₉COOH), myristic acid (C₁₃H₂₇COOH), lauric acid (C₁₁H₂₃COOH), capric acid (C₉H₁₉COOH), caproic acid (C₅H₁₁COOH), oleic acid (C₁₇H₃₃COOH), caprylic acid (C₇H₁₅COOH), erucic acid (C₂₁H₄₁COOH), butyric acid (C₃H₇COOH), ethyl myristate (C₁₃H₂₇COOC₂H₅), ethyl oleate (C₁₇H₃₃COOC₂H₅), ethyl palmitate (C₁₅H₃₁COOC₂H₅), ethyl linoleate (C₁₇H₃₁COOC₂H₅), ethyl laurate (C₁₁H₂₃COOC₂H₅), ethyl linolenate, (C₁₇H₂₉COOC₂H₅), ethyl stearate (C₁₇H₃₅COOC₂H₅), ethyl arachidate (C₁₉H₃₉COOC₂H₅), ethyl caprilate (C₇H₁₅COOC₂H₅), ethyl caprate (C₉H₁₉COOC₂H₅), ethyl caproate (C₅H₁₁COOC₂H₅), ethyl butyrate (C₃H₇COOC₂H₅), triacetin (C₉H₁₄O₆), alpha tocopherol (C₂₉H₅₀O₂), beta tocopherol (C₂₈H₄₈O₂), delta tocopherol (C₂₇H₄₆O₂), gamma tocopherol (C₂₈H₄₈O₂), benzyl alcohol (C₇H₈O), benzyl benzoate (C₁₄H₁₂O₂), methylphenol (C₇H₈O), di-n-butyl sebacate (C₁₈H₃₄O₄), diethylphthalate (C₁₂H₁₄O₄), glyceryl monooleate (C₂₁H₄₀O₄), lecithin, medium chain triglycerides, mineral oil, petrolatum, and liquid paraffines.

In a further embodiment said mixture is divided into a powder component and a liquid component, whereby
A) said powder component comprises the powder component of said two-component bone cement and a polysaccharide in powder form; and
B) said liquid component comprises the liquid component of said two-component bone cement and an aqueous solution of said X-ray contrast agent.

In a further embodiment said third component is a freshly mixed calcium phosphate cement paste.

In a further embodiment the size of all powder particles of said mixture are smaller than 300 micrometers, preferably smaller than 250 micrometers Purposefully the size of at least 80 % of all powder particles is in the range of 50 to 300 micrometers, preferably in the range of 80 to 250 micrometers. This makes the mixture specially suitable for injection into porous bone structures.

The injectable mixture should harden within 7 to 10 minutes, preferably within 8 to 9 minutes after mixing of its components. This keeps time of anesthesia at a minimum and allows immediate patient weight bearing.

Purposefully the hardened mixture has a Young's modulus of elasticity in the range of 10 to 2800 MPa, preferably in the range of 100 to 700 MPa.

The injectable mixture may further comprise an osteoinductive substance, preferably in its third component. Said osteoinductive substance may be chosen from the following group of substances:
a) bone morphogenetic proteins, preferably BMP2, BMP4 or BMP7;
b) growth factors, preferably TGFb-3 (transforming growth factor) or IGF-1 (insulin-like growth factor);
c) plateled-derived growth factor (PDGF);
d) parathyroid hormone (PHT) and parathyroid hormone-related protein (PTHrP);
e) sexual hormones, in particular estrogen; and
prostaglandin.

The injectable mixture may further comprise an antiresorptive substance, preferably in its third component. An antiresorptive substance means a drug, which inhibits resorbtion, i.e. the bone is inhibited to resorb cells. The advantages obtained by the inclusion of such a drug is the possibility of local treatment of osteoporosis which prevents further resorption of the vertebra. Said antiresorptive substance can be a bisphosphonate.

The injectable mixture may further comprise an anabolic substance, a parathyroid hormone (PTH) or an estrogen. An anabolic substance means a drug which generates more bone production, i.e. the bone producing cells are activated.

The injectable mixture may further comprise a hydrogen pump inhibitor, preferably basilomycin A1. The advantage obtained by the inclusion of such a hydrogen pump inhibitor lies in the fact said these drugs are not well applicable systemically and therefore an advantage is obtained by applying them locally.

The injectable bone cement mixture which becomes porous after hardening in situ due to the washing out of its third component is especially useful for treating osteoporosis, for filling bone defects but also as a carrier for an agent for the treatment of osteoporosis.

A possible method for preparing such injectable mixtures for substituting bone tissue in situ may comprise the following steps:
A) the two components of the bone cement are mixed first; and subsequently
B) the obtained mixture is dispersed in the third component.

Another method would comprise the following steps:
A) the two components of the bone cement are mixed first; and subsequently
B) the third component is dispersed in the mixed two-component bone cement.

Still another method would comprise the following steps:
A) Mixing separately a two-component powder/liquid bone cement;
B) Mixing separately a two-component calcium phosphate cement;
C) Adding the separately mixed and still pasty two-component calcium phosphate cement to said separately mixed and still pasty two-component bone cement.

According to a particular embodiment of such methods said third component can be dispersed into the two-component bone cement in such a way that the mean diameter of droplets of the third component dispersed in the two-component bone cement is less than 1 mm, preferably less than 0.5 mm.

The quantity of the injectable mixture to be used for substituting bone tissue in situ depends on the application. In the case of vertebroplasty the quantity is in the range of 2 - 10 ml. In the case of femoroplasty, the injected volumes are very large, namely up to 40 ml. Especially in this latter application the mixture according to invention has the advantage over conventional materials to exhibit a relatively low temperature rise due to the setting reaction.

The invention and additional configurations of the invention are explained in even more detail with reference to the following examples and having reference to the accompanying figures in which:
Fig. 1 shows the mechanical properties of open-porous cylindrical samples of the hardened mixture according to the invention with different amount of aqueous fraction;
Fig. 2 shows the pore size dependence of the open-porous cylindrical samples of the hardened mixture according to the invention on mixing time of the mixture (top left to bottom right: 30s, 60s, 90s, 120s); and

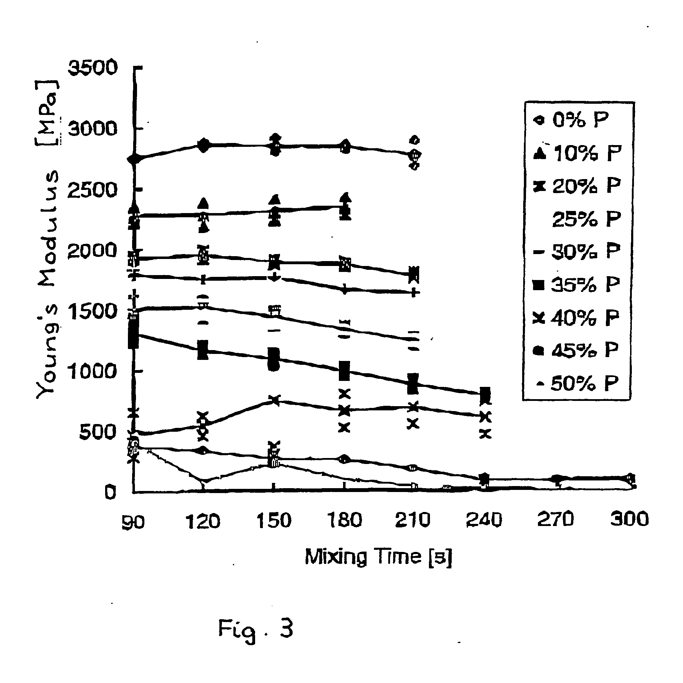

Fig. 3 shows the mixing time dependency of the mechanical properties of the biphasic cylindrical samples and distilled water with 10 weight % hydroxypropylmethylcellulose with different amounts of the aqueous fraction, i.e. porosity (P indication the porosity = aqueous fraction) and with different mixing times

### Example 1 (laboratory)

### a) Composition of the first component

(powder component of the two-component bone cement):
98.2 weight-% of polymethylmethacrylate (PMMA) as filler
1.8 weight-% of benzoyl peroxide as polymerization catalyst

### b) Composition of second component

(liquid component of the two-component bone cement):
98.0 weight-% of methylmethacrylate (MMA) as curing monomer
2.0 weight-% of N,N-dimetyl-p-toluidine as polymerization accelerator

### c) Composition of third component

2 weight-% of hyaluronic acid
98 weight-% of iopromidium as X-ray contrast agent

The porosity of the mixture to be injected is achieved by manual mixing of the highly viscous aqueous fraction represented be the third component to the liquid component (PMMA) of the two-component bone cement. The increased water viscosity is obtained by producing a 2% aqueous solution of hyaluronic acid. The mixing procedure ran in the following manner. Firstly the PMMA powder (powder component of two-component bone cement) and the specific amount of hyaluronic acid (to get a 2% solution) were homogeneously mixed. Than the specific amount of water and - before further mixing - the MMA monomer liquid was added. Manual mixing was done for different durations between 60s and 150s to allow more or less spontaneous phase separation between the acrylic and the aqueous phase during the polymerization process. The porosity was assumed to comply with the aqueous fraction.

Cylindrical samples were produced for the mechanical testing of the modified cement. Commercial 2cc syringes were prepared to serve as cast by cutting off the outflow end. The cement was filled into the syringes by cement injection through a 10cc syringe. The 'casting' syringes were stored vertically during the polymerization for at least 120 min. before pressing out the samples. The environment temperature was 21.5 to 22.0°C. The resulting cylindrical samples had a diameter of 9.54 ± 0.08 mm. The samples were ground within a special adapted steel cast to the length of 16.10 ± 0.09 mm with exactly horizontal tops. The aqueous phase including the whole fraction of the X-ray contrast agent was washed out with water for 60-72h to achieve an open-porous structure of the hardened cement. The samples were stored into water (22.0°C) just until the mechanical testing but not longer than one week.

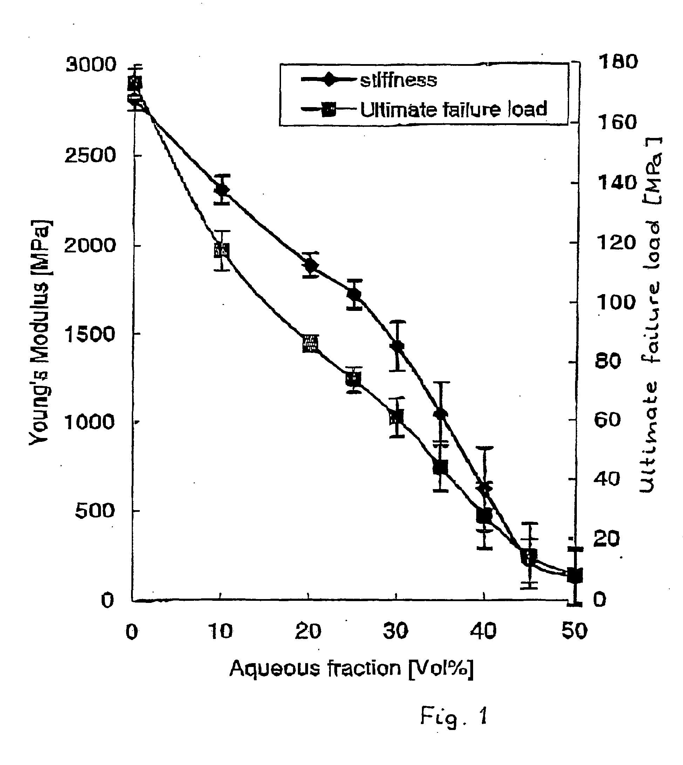

As represented in Fig. 1 the mechanical properties (stiffness measured as Young's Modulus in MPa) and ultimate failure load (measured in MPa) of these samples depend on the amount of the aqueous fraction (in Vol. %).

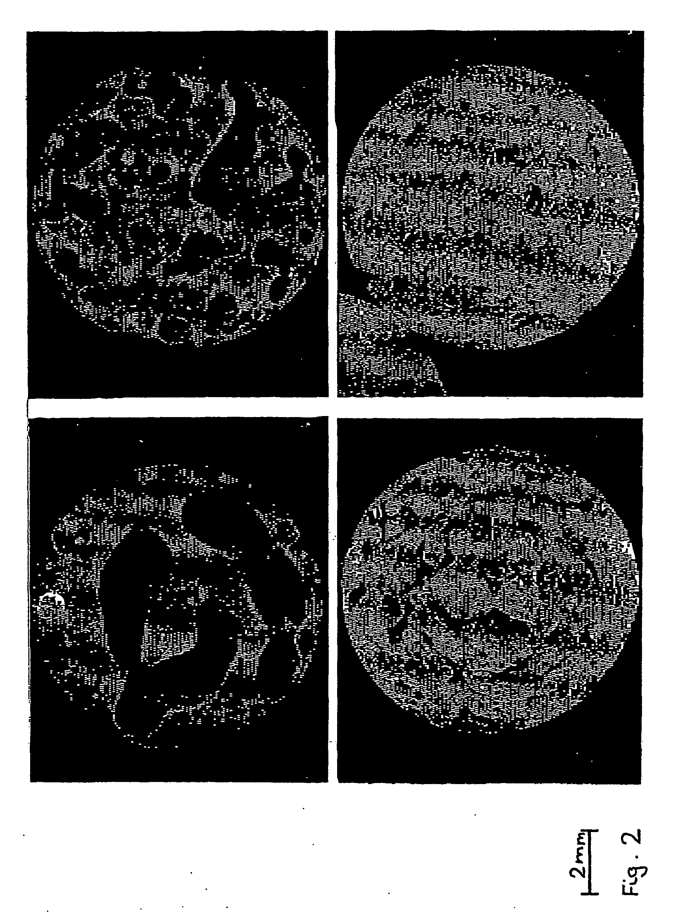

As shown in Fig. 2 the mixing time importantly influences the pore size. The mixing time influences further the mechanical properties (measured as Young's modulus in MPa) of the hardened material. Several graphs for different degrees of porosity (P in %) are represented.

### Example 2 (Clinical application)

The identical material of example 1 was mixed and 10 - 15 ml were injected into the lower thoracic spine of a female cadaver. Injectability, radio-opacity and distribution of the biphasic PMMA-water-compound material were comparable to the commonly used PMMA cements (here Vertebroplastic® , DePuy). A homogenous distribution of the whole compound without any phase-separation was seen microscopically. Mechanical compression testing of the intact (and cement filled) vertebral bodies showed an increased failure load compared to the non-treated vertebrae. However, the stiffness did not increase in the same amount as for unmodified PMMA cements.

### Example 3 (laboratory)

### a) Composition of the first component

(first component of the two-component calcium phosphate cement):
10 g if alpha tricalcium phosphate (Ca₃(PO₄)₂),
0.5 g of Na₂HPO₄
4 ml of water

### b) Composition of second component

(second component of the two-component calcium phosphate cement):
6.5g beta tricalcium phosphate (Ca₃(PO₄)₂),
3.5 g monocalcium phosphatemonohydrate (Ca(H₂PO₄)₂. H₂O)
0.125 g Na₂H₂P₂O₇, and
4 ml of a 0.1M magnesium sulfate solution.

### c) Composition of third component

97 weight-% of Lipiodol® (iodised ethyl ester of the fatty acids of poppy-seed oil).
3 weight-% of hyaluronic acid

## Claims

1. Injectable mixture for substituting bone tissue in situ, said mixture comprising:
A) a two-component powder/liquid bone cement which upon mixing forms a self-hardening cement paste; and
B) a third component consisting of a liquid which essentially is non-miscible with the cement paste and which is suitable to be washed out after hardening of said mixture in situ, resulting in a porous bone substituting material,
**characterized in that**
C) said injectable mixture comprises an X-ray contrast agent which is an organic substance.

2. Mixture according to claim 1, **characterized in that** the X-ray contrast agent is a liquid substance or a solid substance dissolved in a liquid solvent, preferably in water.

3. Mixture according to claim 1 or 2, **characterized in that** the X-ray contrast agent is based on iodine and preferably is chosen from the following group of substances: iopromidum, iopamidol, aminotrizoate acid, iotroxin acid, iopodin acid, iomeprol, iodamid, ioxithalamate, iothalamate, ioxaglin acid and Lipiodol® (iodised ethyl ester of the fatty acids of poppy-seed oil).

4. Mixture according to claim 3, **characterized in that** the iodine-based X-ray contrast agent is used in an aqueous solution, preferably in a concentration of 30 to 80 weight %.

5. Mixture according to one of the claims 1 to 4, **characterized in that** the injectable mixture comprises at least 5 weight %, preferably at least 20 weight % of said X-ray contrast agent.

6. Mixture according to one of the claims 1 to 5, **characterized in that** the viscosity of said third component is lower than 200 Pa.s (200,000 centipoise).

7. Mixture according to claim 6, **characterized in that** said viscosity of said third component is lower than 100 Pa.s (100,000 centipoise), preferably lower than 20 Pa.s (20,000 centipoise).

8. Mixture according to claim 6 or 7, **characterized in that** said viscosity of said third component is between 1 Pa.s and 100 Pa.s (1,000 and 100,000 centipoise) preferably 2 Pa.s and 50 Pa.s (2,000 and 50,000 centipoise)

9. Mixture according to one of the claims 1 to 8, **characterized in that** the viscosity of the injectable mixture measured 4 minutes after mixing of all components is in the range of 200 to 300 Pa.s (200,000 to 300,000 centipoise)

10. Mixture according to one of the claims 1 to 9, **characterized in that** said two-component bone cement is based on a polyacrylic cement, preferably polymethacrylate cement or a calcium phosphate cement.

11. Mixture according to claim 10, **characterized in that** said two-component bone cement is a powder/liquid system base on polymethylmethacrylate (PMMA) powder and monomethylmethacrylate (MMA) liquid with a polymerization catalyst and a polymerization accelerator.

12. Mixture according to one of the claims 1 - 11, **characterized in that** said third component comprises water.

13. Mixture according to one of the claims 1 to 12, **characterized in that** said third component comprises discrete particles of a water-soluble solid substance.

14. Mixture according to claim 13, **characterized in** said water-soluble solid substance is taken from the group of polysaccharides.

15. Mixture according to claim 14, **characterized in** said that polysaccharide is chondroitin sulfate, carboxymethyl cellulose, hydroxyethylmethylcellulose, fucan, carregeenan, dextran, heparin, heparan sulfate, hydroxyethlycellulose (HEC), hydroxypropylmethyl cellulose, sodium alginate, chitosan or a hyaluronate.

16. Mixture according to claim 15, **characterized in** said third component is an aqueous hyaluronate solution.

17. Mixture according to claim 16, **characterized in** said the concentration of said aqueous hyaluronate solution is in the range of 0.1 % to 5.0%, preferably of 1.0 % to 2.0 %.

18. Mixture according to claim 17, **characterized in** said the molecular weight of said hyaluronate is at least 500,000 Daltons, preferably at least 800,000 Daltons.

19. Mixture according to claim 17 or 18, **characterized in** said the molecular weight of said hyaluronate is below 5,000,000 Daltons, preferably below 2,000,000 Daltons.

20. Mixture according to claim 13, **characterized in** said water-soluble solid substance is taken from the group of gelatin or collagen.

21. Mixture according to one of the claims 1 to 20, **characterized in** said third component is a hydrophobic liquid.

22. Mixture according to claim 21, **characterized in** said hydrophobic liquid is selected from the group of : ricinoleic acid (C₁₇H₃₃OCOOH), linoleic acid (C₁₇H₃₁COOH), palmitic acid (C₁₅H₃₁COOH), palmitoleic acid (C₁₅H₂₉COOH), stearic acid (C₁₇H₃₅COOH), linolenic acid (C₁₇H₂₉COOH), arachidic acid (C₁₉H₃₉COOH), myristic acid (C₁₃H₂₇COOH), lauric acid (C₁₁H₂₃COOH), capric acid (C₉H₁₉COOH), caproic acid (C₅H₁₁COOH), oleic acid (C₁₇H₃₃COOH), caprylic acid (C₇H₁₅COOH), erucic acid (C₂₁H₄₁COOH), butyric acid (C₃H₇COOH), ethyl myristate (C₁₃H₂₇COOC₂H₅), ethyl oleate (C₁₇H₃₃COOC₂H₅), ethyl palmitate (C₁₅H₃₁COOC₂H₅), ethyl linoleate (C₁₇H₃₁COOC₂H₅), ethyl laurate (C₁₁H₂₃COOC₂H₅), ethyl linolenate, (C₁₇H₂₉COOC₂H₅), ethyl stearate (C₁₇H₃₅COOC₂H₅), ethyl arachidate (C₁₉H₃₉COOC₂H₅), ethyl caprilate (C₇H₁₅COOC₂H₅), ethyl caprate (C₉H₁₉COOC₂H₅), ethyl caproate (C₅H₁₁COOC₂H₅), ethyl butyrate (C₃H₇COOC₂H₅), triacetin (C₉H₁₄O₆), alpha tocopherol (C₂₉H₅₀O₂), beta tocopherol (C₂₈H₄₈O₂), delta tocopherol (C₂₇H₄₆O₂), gamma tocopherol (C₂₈H₄₈O₂), benzyl alcohol (C₇H₈O), benzyl benzoate (C₁₄H₁₂O₂), methylphenol (C₇H₈O), di-n-butyl sebacate (C₁₈H₃₄O₄), diethylphthalate (C₁₂H₁₄O₄), glyceryl monooleate (C₂₁H₄₀O₄), lecithin, medium chain triglycerides, mineral oil, petrolatum, and liquid paraffines.

23. Mixture according to one of the claims 1 to 22, **characterized in that** it is divided into a powder component and a liquid component, whereby
A) said powder component comprises the powder component of said two-component bone cement and a polysaccharide in powder form; and
B) said liquid component comprises the liquid component of said two-component bone cement and an aqueous solution of said X-ray contrast agent.

24. Mixture according to one of the claims 1 to 23, **characterized in that** the third component is a freshly mixed calcium phosphate cement paste.

25. Mixture according to one of the claims 1 to 24, **characterized in that** the size of all powder particles of the mixture are smaller than 300 micrometers, preferably smaller than 250 micrometers.

26. Mixture according to one of the claims 1 to 25, **characterized in that** the size of at least 80 % of all powder particles is in the range of 50 to 300 micrometers, preferably in the range of 80 to 250 micrometers.

27. Mixture according to one of the claims 1 to 26, **characterized in that** it hardens within 7 to 10 minutes, preferably within 8 to 9 minutes after mixing of its components.

28. Mixture according to one of the claims 1 to 27, **characterized in that** the hardened mixture has a Young's modulus of elasticity in the range of 10 to 2800 MPa, preferably in the range of 100 to 700 MPa.

29. Mixture according to one of the claims 1 to 28, **characterized in that** it further comprises an osteoinductive substance, preferably in its third component.

30. Mixture according to claim 29, **characterized in that** said osteoinductive substance is chosen from the following group of substances:
a) bone morphogenetic proteins, preferably BMP2, BMP4 or BMP7;
b) TGFb-3 (transforming growth factor) or IGF-1 (insulin-like growth factor);
c) plateled-derived growth factor (PDGF);
d) parathyroid hormone (PHT) and parathyroid hormone-related protein (PTHrP);
e) sexual hormones, in particular estrogen; and
f) prostaglandin.

31. Mixture according to one of the claims 1 to 30, **characterized in that** it further comprises an antiresorptive substance, preferably in its third component.

32. Mixture according to claim 31, **characterized in that** the antiresorptive substance is a bisphosphonate.

33. Mixture according to one of the claims 1 to 32, **characterized in that** it further comprises an anabolic substance, a parathyroid hormone (PTH) or an estrogene.

34. Mixture according to one of the claims 1 to 28, **characterized in that** it further comprises a hydrogen pump inhibitor, preferably basilomycin A1.

35. Use of an injectable mixture according to one of the claims 1 to 34 for the manufacture of a medicament for treating osteoporosis.

36. Use of an injectable mixture according to one of the claims 1 to 34 for the manufacture of a medicament for filling bone defects.

37. Use of an injectable mixture according to one of the claims 1 to 34 for the manufacture of a medicament for use as a carrier for an agent for the treatment of osteoporosis.

38. Method for preparing an injectable mixture for substituting bone tissue in situ according to one of the claims 1 to 34 comprising the following steps:
A) the two components of the bone cement are mixed first; and subsequently
B) the obtained mixture is dispersed in the third component.

39. Method for preparing an injectable mixture for substituting bone tissue in situ according to one of the claims 1 to 34 comprising the following steps:
A) the two components of the bone cement are mixed first; and subsequently
B) the third component is dispersed in the mixed two-component bone cement.

40. Method for preparing an injectable mixture for substituting bone tissue in situ according to one of the claims 1 to 34 comprising the following steps:
A) Mixing separately a two-component powder/liquid bone cement;
B) Mixing separately a two-component calcium phosphate cement;
C) Adding the separately mixed and still pasty two-component calcium phosphate cement to said separately mixed and still pasty two-component bone cement.

41. Method according to one of the claims 38 to 40, **characterized in that** said third component is dispersed into the two-component bone cement in such a way that the mean diameter of droplets of the third component dispersed in the two-component bone cement is less than 1 mm, preferably less than 0.5 mm.

## Patentansprüche

1. Injizierbare Mischung als Ersatz von Knochengewebe in situ, genannte Mischung umfasst:
A) Einen pulvrig/flüssigen Zweikomponenten Knochenzement, welcher nach Mischung eine selbsthärtende Zementmasse bildet; und
B) Einer dritten Komponente, welche aus einer Flüssigkeit besteht, die im wesentlichen nicht mit der Zementmasse mischbar ist und welche dazu geeignet ist, nach dem Aushärten genannter Mischung in situ ausgewaschen zu werden, sodass ein poröses Knochenersatzmaterial entsteht,
**dadurch gekennzeichnet, dass**
C) genannte injizierbare Mischung ein Röntgenkontrastmittel enthält, welches eine organische Substanz ist.

2. Mischung gemäss Patentanspruch 1, **dadurch gekennzeichnet, dass** das Röntgenkontrastmittel eine flüssige Substanz oder eine feste, in einem flüssigen Lösungsmittel, bevorzugterweise Wasser, aufgelöste Substanz ist.

3. Mischung gemäss Patentanspruch 1 oder 2, **dadurch gekennzeichnet, dass** das Röntgenkontrastmittel auf Iod basiert und bevorzugterweise aus der folgenden Gruppe von Substanzen ausgewählt ist: lopromidum, lopamidol, Aminotrizoatsäure, lotroxinsäure, lopodinsäure, lomeprol, lodamid, loxithalamat, lothalamat, loxaglinsäure und Lipiodol® (iodierte Ethylester der Fettsäuren aus Mohnöl).

4. Mischung gemäss Patentanspruch 3, **dadurch gekennzeichnet, dass** das auf Iod basierte Röntgenkontrastmittel in einer wässrigen Lösung verwendet wird, bevorzugterweise in einer Konzentration von 30 bis 80 Gewichtsprozent.

5. Mischung gemäss einem der Patentansprüche 1 bis 4, **dadurch gekennzeichnet, dass** die injizierbare Mischung mindestens 5 Gewichtsprozent, bevorzugterweise mindestens 20 Gewichtsprozent des genannten Röntgenkontrastmittels enthält.

6. Mischung gemäss einem der Patentansprüche 1 bis 5, **dadurch gekennzeichnet, dass** die Viskosität der genannten dritten Komponente niedriger als 200 Pa.s (200 000 Centipoise) ist.

7. Mischung gemäss Patentanspruch 6, **dadurch gekennzeichnet, dass** die Viskosität der genannten dritten Komponente niedriger als 100 Pa.s (100 000 Centipoise), bevorzugterweise niedriger als 20 Pa.s (20 000 Centipoise) ist.

8. Mischung gemäss Patentanspruch 6 oder 7, **dadurch gekennzeichnet, dass** die Viskosität der genannten dritten Komponente zwischen 1 und 100 Pa.s (1 000 und 100 000 Centipoise), bevorzugterweise zwischen 2 und 50 Pa.s (2 000 and 50 000 Centipoise) ist.

9. Mischung gemäss einem der Patentansprüche 1 bis 8, **dadurch gekennzeichnet, dass** die 4 Minuten nach der Mischung aller Komponenten gemessene Viskosität der injizierbaren Mischung im Bereich von 200 bis 300 Pa.s (200 00 bis 300 00 Centipoise) liegt.

10. Mischung gemäss einem der Patentansprüche 1 bis 9, **dadurch gekennzeichnet, dass** der genannte Zweikomponenten-Knochenzement auf Polyacryl-Zement basierend ist, bevorzugterweise Polymethacrylat-Zement oder ein Calciumphosphatzement.

11. Mischung gemäss Patentanspruch 10, **dadurch gekennzeichnet, dass** der genannte Zweikomponenten-Knochenzement ein pulvrig/flüssiges System basierend auf Polymethylmethacrylat (PMMA)-Pulver und Monomethylmethacrylat (MMA) Flüssigkeit mit einem Polymerisations-Katalysator und einem PolymerisationsBeschleuniger ist.

12. Mischung gemäss einem der Patentansprüche 1 - 11, **dadurch gekennzeichnet, dass** die genannte dritte Komponente Wasser enthält.

13. Mischung gemäss einem der Patentansprüche 1 bis 12, **dadurch gekennzeichnet, dass** die genannte dritte Komponente diskrete Partikel einer wasserlöslichen festen Substanz enthält.

14. Mischung gemäss Patentanspruch 13, **dadurch gekennzeichnet, dass** die genannte wasserlösliche feste Substanz aus der Gruppe der Polysaccharide stammt.

15. Mischung gemäss Patentanspruch 14, **dadurch gekennzeichnet, dass** das genannte Polysaccharid Chondroitinsulfat, Carboxymethylcellulose, Hydroxyethylmethylcellulose, Fucan, Carrageneen, Dextran, Heparin, Heparansulfat, Hydroxyethlycellulose (HEC), Hydroxypropylmethylcellulose, Natriumalginat, Chitosan oder ein Hyaluronat ist.

16. Mischung gemäss Patentanspruch 15, **dadurch gekennzeichnet, dass** die genannte dritte Komponente eine wässrige Hyaluronat-Lösung ist.

17. Mischung gemäss Patentanspruch 16, **dadurch gekennzeichnet, dass** die Konzentration der genannten wässrigen Hyaluronat-Lösung im Bereich von 0.1 % bis 5.0%, bevorzugterweise von 1.0 % bis 2.0 % liegt.

18. Mischung gemäss Patentanspruch 17, **dadurch gekennzeichnet, dass** das Molekulargewicht des genannten Hyaluronats mindestens 500 000 Dalton, bevorzugterweise mindestens 800 000 Dalton beträgt.

19. Mischung gemäss Patentanspruch 17 oder 18, **dadurch gekennzeichnet, dass** das Molekulargewicht des genannten Hyaluronats niedriger als 5 000 000, bevorzugterweise niedriger als 2 000 000 Dalton ist.

20. Mischung gemäss Patentanspruch 13, **dadurch gekennzeichnet, dass** die genannte wasserlösliche Substanz aus der Gruppe von Gelatine oder Collagen stammt.

21. Mischung gemäss einem der Patentansprüche 1 - 20, **dadurch gekennzeichnet, dass** die genannte dritte Komponente eine hydrophobe Flüssigkeit ist.

22. Mischung gemäss Patentanspruch 21, **dadurch gekennzeichnet, dass** die genannte hydrophobe Flüssigkeit aus der folgenden Gruppe gewählt wurde: Ricinolsäure (C₁₇H₃₃OCOOH), Linolsäure (C₁₇H₃₁COOH), Palmitinsäure (C₁₅H₃₁COOH), Palmitoleinsäure (C₁₅H₂₉COOH), Stearinsäure (C₁₇H₃₅COOH), Linolensäure (C₁₇H₂₉COOH), Arachidsäure (C₁₉H₃₉COOH), Myristinsäure (C₁₃H₂₇COOH), Laurinsäure (C₁₁H₂₃COOH), Caprinsäure (C₉H₁₉COOH), Capronsäure (C₅H₁₁COOH), Oelsäure (C₁₇H₃₃COOH), Caprylsäure (C₇H₁₅COOH), Erucasäure (C₂₁H₄₁COOH), Buttersäure (C₃H₇COOH), Ethylmyristat (C₁₃H₂₇COOC₂H₅), Ethyloleat (C₁₇H₃₃COOC₂H₅), Ethylpalmitat (C₁₅H₃₁COOC₂H₅), Ethyllinoleat (C₁₇H₃₁COOC₂H₅), Ethyllaurat (C₁₁H₂₃COOC₂H₅), Ethyllinolenat, (C₁₇H₂₉COOC₂H₅), Ethylstearat (C₁₇H₃₅COOC₂H₅), Ethylarachidat (C₁₉H₃₉COOC₂H₅), Ethylcaprilat (C₇H₁₅COOC₂H₅), Ethylcaprat (C₉H₁₉COOC₂H₅), Ethylcaproat (C₅H₁₁COOC₂H₅), Ethylbutyrat (C₃H₇COOC₂H₅), Triacetin (C₉H₁₄O₆), alpha Tocopherol (C₂₉H₅₀O₂), beta Tocopherol (C₂₈H₄₈O₂), delta Tocopherol (C₂₇H₄₆O₂), gamma Tocopherol (C₂₈H₄₈O₂), Benzylalkohol (C₇H₈O), Benzylbenzoat (C₁₄H₁₂O₂), Methylphenol (C₇H₈O), di-n-Butylsebacat (C₁₈H₃₄O₄), Diethylphthalat (C₁₂H₁₄O₄), Glycerylmonooleat (C₂₁H₄₀O₄), Lecithin, Triglyceride mittlerer Kettenlänge, Mineralöl, Petrolatum, und flüssige Paraffine.

23. Mischung gemäss einem der Patentansprüche 1 bis 22, **dadurch gekennzeichnet, dass** sie in eine pulvrige Komponente und eine flüssige Komponente getrennt ist, wobei
A) die genannte pulvrige Komponente die pulvrige Komponente des genannten Zweikomponenten-Knochenzements und ein Polysaccharid in Pulverform enthält; und
B) die genannte flüssige Komponente die flüssige Komponente des genannten Zweikomponenten-Knochenzements und eine wässrige Lösung des genannten Röntgenkontrastmittels enthält.

24. Mischung gemäss einem der Patentansprüche 1 bis 23, **dadurch gekennzeichnet, dass** die dritte Komponente eine frisch gemischte Calciumphosphat-Zementmasse ist.

25. Mischung gemäss einem der Patentansprüche 1 bis 24, **dadurch gekennzeichnet, dass** die Grösse aller Pulverpartikel der Mischung kleiner als 300 Mikrometer, bevorzugterweise kleiner als 250 Mikrometer ist.

26. Mischung gemäss einem der Patentansprüche 1 bis 25, **dadurch gekennzeichnet, dass** die Grösse von mindestens 80 % aller Pulverpartikel im Bereich zwischen 50 bis 300 Mikrometer, bevorzugterweise im Bereich zwischen 80 und 250 Mikrometer liegt.

27. Mischung gemäss einem der Patentansprüche 1 bis 26, **dadurch gekennzeichnet, dass** sie innerhalb von 7 bis 10 Minuten, bevorzugterweise innerhalb von 8 bis 9 Minuten, nach der Mischung ihrer Komponenten aushärtet.

28. Mischung gemäss einem der Patentansprüche 1 bis 27, **dadurch gekennzeichnet, dass** die ausgehärtete Mischung ein Elastizitätsmodul nach Young im Bereich von 10 bis 2800 MPa, bevorzugterweise im Bereich von 100 bis 700 Mpa hat.

29. Mischung gemäss einem der Patentansprüche 1 bis 28, **dadurch gekennzeichnet, dass** sie ausserdem eine osteoinduktive Substanz, bevorzugterweise in ihrer dritten Komponente, enthält.

30. Mischung gemäss Patentanspruch 29, **dadurch gekennzeichnet, dass** die genannte osteoinductive Substanz aus der folgenden Gruppe von Substanzen ausgewählt ist:
a) "Bone Morphogenetic Proteins", bevorzugterweise BMP2, BMP4 oder BMP7;
b) TGFbeta-3 (Transforming Growth Factor) oder IGF-1 (Insulin-like Growth Factor);
c) "Platelet-Derived Growth Factor (PDGF)";
d) Parathormon (PHT) und "Parathyroid Hormone-related Protein" (PTHrP);
e) Geschlechtshormone, insbesondere Oestrogen; und
a) Prostaglandin.

31. Mischung gemäss einem der Patentansprüche 1 bis 30, **dadurch gekennzeichnet, dass** sie ausserdem eine antiresorptive Substanz, bevorzugterweise in ihrer dritten Komponente, enthält.

32. Mischung gemäss Patentanspruch 31, **dadurch gekennzeichnet, dass** die antiresorptive Substanz ein Bisphosphonat ist.

33. Mischung gemäss einem der Patentansprüche 1 bis 32, **dadurch gekennzeichnet, dass** sie ausserdem eine anabole Substanz, ein Parathormon (PTH) oder ein Oestrogen enthält.

34. Mischung gemäss einem der Patentansprüche 1 bis 28, **dadurch gekennzeichnet, dass** sie ausserdem einen Protonenpumpenhemmer, bevorzugterweise Basilomycin A1, enthält.

35. Verwendung einer injizierbaren Mischung gemäss einem der Patentansprüche 1 bis 34 zur Herstellung eines Medikamentes zur Behandlung von Osteoporose.

36. Verwendung einer injizierbaren Mischung gemäss einem der Patentansprüche 1 bis 34 zur Herstellung eines Medikamentes um Knochendefekt auszufüllen.

37. Verwendung einer injizierbaren Mischung gemäss einem der Patentansprüche 1 bis 34 zur Herstellung eines Medikamentes zur Verwendung als Trägermaterial für ein Medikament zur Behandlung von Osteoporose.

38. Methode zur Herstellung einer injizierbaren Mischung zum Ersatz von Knochengewebe in situ gemäss einem der Patentansprüche 1 bis 34 mit folgenden Schritten:
A) Die beiden Komponenten des Knochenzements werden zuerst gemischt, und danach wird
B) die erhaltene Mischung in der dritten Komponente dispergiert.

39. Methode zur Herstellung einer injizierbaren Mischung zum Ersatz von Knochengewebe in situ gemäss einem der Patentansprüche 1 bis 34 mit folgenden Schritten:
A) Die beiden Komponenten des Knochenzements werden zuerst gemischt, und danach wird
B) die dritte Komponente in dem gemischten Zweikomponenten-Knochenzement dispergiert wird.

40. Methode zur Herstellung einer injizierbaren Mischung zum Ersatz von Knochengewebe in situ gemäss einem der Patentansprüche 1 bis 34 mit folgenden Schritten:
A) Getrenntes Mischen eines pulvrig/flüssigen Zweikomponenten-Knochenzementes;
B) Getrenntes Mischen eines Zweikomponenten Calciumphosphat-Zementes;
C) Zugeben des getrennt gemischten und noch pastösen Zweikomponenten-Calciumphosphat-Zementes zu dem genannten, getrennt gemischten und noch pastösen Zweikomponenten-Knochenzement zugeben.

41. Methode gemäss einem der Patentansprüche 38 bis 40, **dadurch gekennzeichnet, dass** die genannte dritte Komponente in dem Zweikomponenten-Knochenzement auf eine solche Art dispergiert wird, dass der mittlere Durchmesser der Tröpfchen der in dem Zweikomponenten-Knochenzement dispergierten dritten Komponente weniger als 1 mm, bevorzugterweise weniger als 0.5 mm ist.

## Revendications

1. Mélange injectable pour remplacer un tissu osseux *in situ*, ledit mélange comprenant :
A) un ciment osseux à deux composants poudre/liquide qui, lors du mélange, forme une pâte de ciment auto-durcissant ; et
B) un troisième composant constitué par un liquide qui est essentiellement non miscible avec la pâte de ciment et qui est approprié pour partir au lavage après durcissement dudit mélange *in situ*, aboutissant à une matière poreuse de remplacement d'os,
**caractérisé en ce que**
C) ledit mélange injectable comprend un agent de contraste aux rayons X qui est une substance organique.

2. Mélange selon la revendication 1, **caractérisé en ce que** l'agent de contraste aux rayons X est une substance liquide ou une substance solide dissoute dans un solvant liquide, de préférence dans l'eau.

3. Mélange selon la revendication 1 ou 2, **caractérisé en ce que** l'agent de contraste aux rayons X est à base d'iode et de préférence est choisi dans le groupe suivant de substances : l'iopromidum, l'iopamidol, l'acide aminotrizoate, l'acide iotroxinique, l'acide iopodinique, l'ioméprol, l'iodamide, l'ioxithalamate, l'iothalamate, l'acide ioxaglinique et Lipiodol® (ester éthylique iodé des acides gras d'huile de graine de pavot).

4. Mélange selon la revendication 3, **caractérisé en ce que** l'agent de contraste aux rayons X à base d'iode est utilisé dans une solution aqueuse, de préférence dans une concentration de 30 à 80% en poids.

5. Mélange selon l'une quelconque des revendications 1 à 4, **caractérisé en ce que** le mélange injectable comprend au moins 5% en poids, de préférence au moins 20% en poids dudit agent de contraste aux rayons X.

6. Mélange selon l'une quelconque des revendications 1 à 5, **caractérisé en ce que** la viscosité dudit troisième composant est inférieure à 200 Pa.s (200 000 centipoises).

7. Mélange selon la revendication 6, **caractérisé en ce que** ladite viscosité dudit troisième composant est inférieure à 100 Pa.s (100 000 centipoises), de préférence inférieure à 20 Pa.s (20 000 centipoises).

8. Mélange selon la revendication 6 ou 7, **caractérisé en ce que** ladite viscosité dudit troisième composant est comprise entre 1 et 100 Pa.s (1 000 et 100 000 centipoises), de préférence 2 et 50 Pa.s (2 000 et 50 000 centipoises).

9. Mélange selon l'une quelconque des revendications 1 à 8, **caractérisé en ce que** la viscosité du mélange injectable mesurée 4 minutes après le mélange de tous les composants est dans la gamme allant de 200 à 300 Pa.s (200 000 à 300 000 centipoises).

10. Mélange selon l'une quelconque des revendications 1 à 9, **caractérisé en ce que** ledit ciment osseux à deux composants est à base de ciment polyacrylique, de préférence le ciment de polyméthacrylate ou un ciment de phosphate de calcium.

11. Mélange selon la revendication 10, **caractérisé en ce que** ledit ciment osseux à deux composants est un système poudre/liquide basé sur la poudre de poly(méthacrylate de méthyle) (PMMA) et le liquide de mono(méthacrylate de méthyle) (MMA) avec un catalyseur de polymérisation et un accélérateur de polymérisation.

12. Mélange selon l'une quelconque des revendications 1 à 11, **caractérisé en ce que** ledit troisième composant comprend de l'eau.

13. Mélange selon l'une quelconque des revendications 1 à 12, **caractérisé en ce que** ledit troisième composant comprend des particules discrètes d'une substance solide hydrosoluble.

14. Mélange selon la revendication 13, **caractérisé en ce que** ladite substance solide hydrosoluble est prise dans le groupe des polysaccharides.

15. Mélange selon la revendication 14, **caractérisé en ce que** ledit polysaccharide est le sulfate de chondroïtine, la carboxyméthylcellulose, l'hydroxyéthylméthylcellulose, le fucan, la carragénine, le dextrane, l'héparine, le sulfate d'héparane, l'hydroxyéthylcellulose (HEC), l'hydroxypropylméthylcellulose, l'alginate de sodium, le chitosane ou un hyaluronate.

16. Mélange selon la revendication 15, **caractérisé en ce que** ledit troisième composant est une solution aqueuse d'hyaluronate.

17. Mélange selon la revendication 16, **caractérisé en ce que** ladite concentration de ladite solution aqueuse d'hyaluronate est dans la gamme allant de 0,1 % jusqu'à 5,0%, de préférence de 1,0% à 2,0%.

18. Mélange selon la revendication 17, **caractérisé en ce que** ladite masse moléculaire dudit hyaluronate est d'au moins 500 000 Daltons, de préférence au moins 800 000 Daltons.

19. Mélange selon la revendication 17 ou 18, **caractérisé en ce que** ladite masse moléculaire dudit hyaluronate est en dessous de 5 000 000 Daltons, de préférence en dessous de 2 000 000 Daltons.

20. Mélange selon la revendication 13, **caractérisé en ce que** ladite substance solide hydrosoluble est prise dans le groupe formé par la gélatine ou le collagène.

21. Mélange selon l'une quelconque des revendications 1 à 20, **caractérisé en ce que** ledit troisième composant est un liquide hydrophobe.

22. Mélange selon la revendication 21, **caractérisé en ce que** ledit liquide hydrophobe est choisi dans le groupe formé par : l'acide ricinoléique (C₁₇H₃₃OCOOH), l'acide linoléique (C₁₇H₃₁COOH), l'acide palmitique (C₁₅H₃₁COOH), l'acide palmitoléique (C₁₅H₂₉COOH), l'acide stéarique (C₁₇H₃₅COOH), l'acide linolénique (C₁₇H₂₉COOH), l'acide arachidique (C₁₉H₃₉COOH), l'acide myristique (C₁₃H₂₇COOH), l'acide laurique (C₁₁H₂₃COOH), l'acide caprique (C₉H₁₉COOH), l'acide caproïque (C₅H₁₁COOH), l'acide oléique (C₁₇H₃₃COOH), l'acide caprylique (C₇H₁₅COOH), l'acide érucique (C₂₁H₄₁COOH), l'acide butyrique (C₃H₇COOH), le myristate d'éthyle (C₁₃H₂₇COOC₂H₅), l'oléate d'éthyle (C₁₇H₃₃COOC₂H₅), le palmitate d'éthyle (C₁₅H₃₁COOC₂H₅), le linoléate d'éthyle (C₁₇H₃₁COOC₂H₅), le laurate d'éthyle (C₁₁H₂₃COOC₂H₅), le linolénate d'éthyle, (C₁₇H₂₉COOC₂H₅), le stéarate d'éthyle (C₁₇H₃₅COOC₂H₅), l'arachidate d'éthyle (C₁₉H₃₉COOC₂H₅), le caprilate d'éthyle (C₇H₁₅COOC₂H₅), le caprate d'éthyle (C₉H₁₉COOC₂H), le caproate d'éthyle (C₅H₁₁COOC₂H₅), le butyrate d'éthyle (C₃H₇COOC₂H₅), la triacétine (C₉H₁₄O₆), alpha tocophérol (C₂₉H₅₀O₂), le bêta tocophérol (C₂₈H₄₈O₂), le delta tocophérol (C₂₇H₄₆O₂), le gamma tocophérol (C₂₈H₄₈O₂), l'alcool benzylique (C₇H₈O), le benzoate de benzyle (C₁₄H₁₂O₂), le méthylphénol (C₇H₈O), le sébacate de di-n-butyle (C₁₈H₃₄O₄), le phtalate de diéthyle (C₁₂H₁₄O₄), le mono-oléate de glycéryle (C₂₁H₄₀O₄), la lécithine, les triglycérides à chaîne moyenne, l'huile minérale, le pétrolatum, et les paraffines liquides.

23. Mélange selon l'une quelconque des revendications 1 à 22, **caractérisé en ce qu'**il est divisé en un composant en poudre et en un composant liquide, ainsi
A) ledit composant en poudre comprend le composant en poudre dudit ciment osseux à deux composants et un polysaccharide sous forme de poudre ; et
B) ledit composant liquide comprend le composant liquide dudit ciment osseux à deux composants et une solution aqueuse dudit agent de contraste aux rayons X.

24. Mélange selon l'une quelconque des revendications 1 à 23, **caractérisé en ce que** le troisième composant est une pâte de ciment de phosphate de calcium fraîchement mélangée.

25. Mélange selon l'une quelconque des revendications 1 à 24, **caractérisé en ce que** la taille de toutes les particules de poudre du mélange est inférieure à 300 micromètres, de préférence inférieure à 250 micromètres.

26. Mélange selon l'une quelconque des revendications 1 à 25, **caractérisé en ce que** la taille d'au moins 80% de toutes les particules de poudre est dans la gamme allant de 50 à 300 micromètres, de préférence dans la gamme allant de 80 à 250 micromètres.

27. Mélange selon l'une quelconque des revendications 1 à 26, **caractérisé en ce qu'**il durcit en 7 à 10 minutes, de préférence en 8 à 9 minutes après mélange de ses composants.

28. Mélange selon l'une quelconque des revendications 1 à 27, **caractérisé en ce que** le mélange durci présente un module d'élasticité de Young dans la gamme allant de 10 à 2 800 MPa, de préférence dans la gamme allant de 100 à 700 MPa.

29. Mélange selon l'une quelconque des revendications 1 à 28, **caractérisé en ce qu'**il comprend en outre une substance ostéoinductive, de préférence dans son troisième composant.

30. Mélange selon la revendication 29, **caractérisé en ce que** ladite substance ostéoinductive est choisie dans le groupe de substances suivant :
a) les protéines morphogénétiques osseuses, de préférence BMP2, BMP4 ou BMP7 ;
b) TGFb-3 (facteur de croissance transformant) ou IGF-1 (somatomédine) ;
c) facteur de croissance dérivé des plaquettes (PDGF) ;
d) hormone parathyroïde (PHT) et la protéine apparentée à l'hormone parathyroïde (PTHrP) ;
e) les hormones sexuelles, en particulier l'oestrogène ; et
f) la prostaglandine.

31. Mélange selon l'une quelconque des revendications 1 à 30, **caractérisé en ce qu'**il comprend en outre une substance antirésorption, de préférence dans son troisième composant.

32. Mélange selon la revendication 31, **caractérisé en ce que** la substance antirésorption est un bisphosphonate.

33. Mélange selon l'une quelconque des revendications 1 à 32, **caractérisé en ce qu'**il comprend en outre une substance anabolique, une hormone parathyroïde (PTH) ou un estrogène.

34. Mélange selon l'une quelconque des revendications 1 à 28, **caractérisé en ce qu'**il comprend en outre un inhibiteur de la pompe à hydrogène, de préférence la basilomycine A1.

35. Utilisation d'un mélange injectable selon l'une quelconque des revendications 1 à 34 pour la fabrication d'un médicament en vue de traiter l'ostéoporose.

36. Utilisation d'un mélange injectable selon l'une quelconque des revendications 1 à 34 pour la fabrication d'un médicament en vue de remplir les défauts osseux.

37. Utilisation d'un mélange injectable selon l'une quelconque des revendications 1 à 34 pour la fabrication d'un médicament pour une utilisation en tant que véhicule pour un agent en vue du traitement de l'ostéoporose.

38. Procédé de préparation d'un mélange injectable en vue de remplacer un tissu osseux *in situ* selon l'une quelconque des revendications 1 à 34 comprenant les étapes suivantes :
A) les deux composants du ciment osseux sont mélangés dans un premier temps ; et ultérieurement
B) le mélange obtenu est dispersé dans le troisième composant.

39. Procédé de préparation d'un mélange injectable en vue de remplacer un tissu osseux *in situ* selon l'une quelconque des revendications 1 à 34 comprenant les étapes suivantes :
A) les deux composants du ciment osseux sont mélangés dans un premier temps ; et ultérieurement
B) le troisième composant est dispersé dans le ciment osseux mélangé à deux composants.

40. Procédé de préparation d'un mélange injectable en vue de remplacer un tissu osseux *in situ* selon l'une quelconque des revendications 1 à 34 comprenant les étapes suivantes consistant à :
A) Mélanger séparément un ciment osseux à deux composants poudre/liquide ;
B) Mélanger séparément un ciment de phosphate de calcium à deux composants ;
C) Ajouter le ciment de phosphate de calcium à deux composants encore pâteux et mélangé séparément audit ciment osseux à deux composants encore pâteux et mélangé séparément.

41. Procédé selon l'une quelconque des revendications 38 à 40, **caractérisé en ce que** ledit troisième composant est dispersé dans le ciment osseux à deux composants de sorte que le diamètre moyen des gouttelettes du troisième composant dispersé dans le ciment osseux à deux composants est inférieur à 1 mm, de préférence inférieur à 0,5 mm.
